(19) Europäisches Patentamt
European Patent Office
Office européen des brevets

(11) Publication number: 0 260 778
A1

# (12) EUROPEAN PATENT APPLICATION

(21) Application number: **87301254.6**

(22) Date of filing: **13.02.87**

(51) Int. Cl.⁴: **C 12 P 37/00**, C 12 P 35/00, C 07 K 5/06 // C12R1/465, C12R1/75, C12R1/82

(30) Priority: **04.09.86 US 903548**

(43) Date of publication of application: **23.03.88 Bulletin 88/12**

(84) Designated Contracting States: **AT BE CH DE ES FR GB GR IT LI NL SE**

(71) Applicant: **THE CHANCELLOR, MASTERS AND SCHOLARS OF THE UNIVERSITY OF OXFORD, Wellington Square, Oxford OX1 2JD (GB)**

(72) Inventor: **Baldwin,Jack Edward, 10 Rolfe Place, Headington, Oxford (GD)**

(74) Representative: **Hudson, Christopher Mark, Erl Wood Manor, Windlesham Surrey GU20 6PH (GB)**

(54) **Enzymatic process for B-Lactams.**

(57) N-(3-Carboxyphenylacetyl)-L-cysteinyl-D-valine and N-(3--carboxyphenylacetyl)-L-cysteinyl-D-modified-valine dipeptides are substrates for isopenicillin N synthetase conversion to penams e.g. 2-vinyl-2-methylpenam, 2-allenylpenam and 2-methoxypenam. The substitution of the 3-carboxyphenylacetyl group for the 2-α-aminoadipoyl group of the natural tripeptide L-AAA-L-cys-D-val and tripeptides with modified valine affords efficient conversion to the penams.

EP 0 260 778 A1

ENZYMATIC PROCESS FOR β-LACTAMS

This invention relates to a process for preparing β-lactam antibiotics. In particular it relates to an enzymatic process for preparing penicillin and cephalosporin antibiotics.

The enzyme, isopenicillin N synthetase ("cyclase," IPNS) is well known for its ability to cyclize 2-(L-α-aminoadipoyl)-L-cysteinyl-D-valine to isopenicillin N. Studies by J.E. Baldwin et al., J. Chem. Soc., Chem. Commun., 1984, 1211, with the purified enzyme have shown that the enzyme is capable of forming the cepham ring system as well as the penam system.

According to the present invention there is provided a process for preparing a β-lactam of formula 1

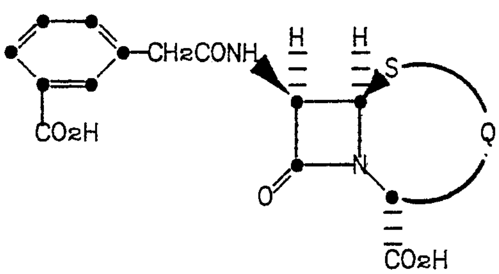

1

where Q represents a group of formula

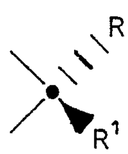

or

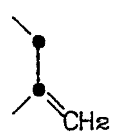

and

wherein R and $R_1$ independently are hydrogen or $C_1$-$C_4$ alkyl; or one of R and $R_1$ is hydrogen or $C_1$-$C_4$ alkyl and the other is $C_1$-$C_3$ alkoxy, $C_2$-$C_4$ alkenyl or allenyl. The process comprises contacting in an aqueous medium at a pH of between about 6 and about 9 in the presence of oxygen, ferrous ion and ascorbic acid, a dipeptide represented by the formula 2,

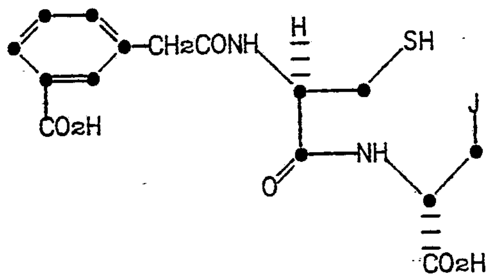

2

where J represents

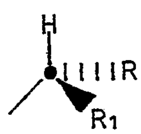

or

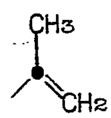

wherein R and $R_1$ have the above defined meanings, with the enzyme isopenicillin N synthetase.

The term $C_1$-$C_4$ alkyl as used herein refers to methyl, ethyl, n-propyl, isopropyl, n-butyl, sec-butyl, isobutyl and t-butyl; and $C_1$-$C_3$ alkoxy refers to methoxy, ethoxy, n-propoxy and isopropoxy. The term allenyl refers to the propadiene group $CH_2$=C=CH-.

The isopenicillin N synthetase used in the process can be obtained from numerous sources including *Cephalosporium acremonium*, such as *C. acremonium* ATCC 48272 and ATCC 36225, *Penicillium chrysogenum*, *Aspergillus nidulans*, *Streptomyces clavuligerus* ATCC 27064, and *Streptomyces lipanii*, ATCC 27357. The enzyme is used in the form of a cell-free extract in a semi-purified form or, preferably, as the purified enzyme, e.g., prepared as described by Pang et al., *Biochem J.* (1984), *222*, 789-795, and by J.E. Baldwin et al., *FEBS Letter*, 1985, *188*, 253. A preferred source of the enzyme is *Cephalosporium acremonium*.

The dipeptide substrates represented by the formula 2 can be prepared by known coupling methods. For example, an S-protected, carboxy-protected L-cysteinyl-D-valine or L-cysteinyl-D-modified-valine is acylated with 3-(protected-carboxy)phenylacetic acid via the EEDQ method and the protecting groups removed.

The term "modified valine" is used herein for convenience to designate a variety of amino acids represented by the formula 3

$$H_2N-\underset{\displaystyle CO_2H}{\underset{|}{CH}}-\overset{\displaystyle R}{\overset{|}{CH}}-R_1 \qquad 3$$

which are coupled with cysteine to form the dipeptide precursors of the substrates represented by the formula 2. These modified-valine amino acids replace the valine of the natural tripeptide and, in a broad

sense, are modifications of valine.

Examples of modified valines used to prepare the dipeptides are D-isoleucine, D-norvaline, D-serine O-methyl ether, D-serine O-ethyl ether, 2-amino-3-methoxybutanoic acid, 2-amino-3-isopropoxybutanoic acid, 2-amino-4,5-hexadienoic acid, 2-amino-3-methyl-4,5-hexadienoic acid, 2-aminobutanoic acid, 2-amino-3-methylpentanoic acid, 2-amino-3-ethylpentanoic acid, 2-amino-3-methylpent-4-enoate, and 2-amino-3-ethylpent-4-enoate.

The "modified valines" exemplified above are prepared by known methods or variations thereof as shown in the preparative Examples which follow herein.

The dipeptide substrate, formula 2, is generally employed in the process at a concentration of between about 0.1 mM and about 5 mM. The substrate should be relatively pure and free of contamination e.g. by heavy metals that may inhibit the enzyme.

The IPNS enzyme is used in large excess relative to the substrate for best results. The activity of the enzyme to be used in the process can be measured by the assay described by Pang et al., supra. The activity is expressed in terms of units with one unit of activity equal to the amount of enzyme required to form 1 μmol of isopenicillin N per minute with the natural tripeptide (L-α-amino-δ-adipoyl)-L-cysteinyl-D-valine.

The process is carried out in the presence of ferrous ion and ascorbic acid (preferably L-ascorbic acid). Depending on the pH, the ascorbic acid may be

in the form of ascorbate. These cofactors enhance the activity of the enzyme. The minimum amount of ferrous ion required to activate the enzyme is used. Generally, the concentration of ferrous ion is between about 50 μM to about 0.2 mM. The higher the purity of the enzyme the lower the amount of ferrous ion required for maximum activation. In general the L-ascorbic acid, which is used in conjunction with ferrous ion, is employed at a concentration about equal to the $Fe^{2+}$ concentration although higher concentrations may be used. Sources of ferrous ion are the salts, ferrous sulfate, ferrous chloride, ferrous carbonate, or other suitable salt.

The process is carried out in the presence of oxygen. On a small scale e.g. laboratory size, sufficient oxygen can be supplied by carrying out the process in an open vessel. On a large scale, particularly when a large excess of enzyme is used, oxygen may be supplied by passing air or oxygen into the incubation mixture. During the process the incubation mixture is agitated well by stirring or shaking.

During the process hydrogen peroxide is generated. To avoid excess levels of peroxide, which may be deleterious to the enzyme, the substrate, or the product, a catalase such as beef liver catalase can be added to the incubation mixture.

The process is carried out at a temperature between about 20°C and about 40°C and preferably between about 25°C and about 30°C. Best yields have been obtained at a temperature of about 27°C with the N-(3-carboxyphenylacetyl)-L-cysteinyl-D-valine substrate.

The process is carried out at a pH of between about 6 and about 9 and preferably at between about pH 7.5 and 8.5. The incubation mixture can be maintained at the desired pH with a buffer such as ammonium carbonate, tris buffer, or MOPS buffer, 3-(N-morpholino)-propanesulfonic acid.

A suitable reducing agent is added to the incubation mixture during the process. Since the process is an oxidative process the reducing agent apparently serves to prevent or retard disulfide formation by the cysteinyl sulfhydryl group. The reducing agent also may be effective in preventing oxidation of the ferrous ion to ferric ion. The latter is a far less effective IPNS stimulant than is ferrous ion.

The formation of the penicillin proceeds rapidly under the above described process conditions when purified enzyme is used in excess with substrate of high purity. On laboratory scale the process can be carried out for a period of from about 10 minutes to about 2 hours. Large scale incubations of enzyme and substrate can require somewhat longer times.

The process is terminated by the addition of a solvent such as acetone or ethyl alcohol to the incubation mixture. The precipitated protein is separated, for example by centrifugation, and the product is isolated from the supernatant by conventional means. The aqueous liquid phase containing the product can be lyophilized and the product isolated by chromatography such as by reverse phase octadecylsilane HPLC. Alternatively the product penicillin may be isolated by extraction of the incubation mixture following separation of protein.

In a preferred embodiment of the process, N-(3-carboxyphenylacetyl)-L-cysteinyl-D-valine is incubated with excess purified IPNS enzyme to form 6-(3-carboxyphenylacetylamino)-2,2-dimethylpenam-3-carboxylic acid.

Examples of penicillins provided by the invention are 6-(3-carboxyphenylacetylamino)-2α-vinyl-2β-methylpenam-3-carboxylic acid,

6-(3-carboxyphenylacetylamino)-2,2-diethylpenam-3-carboxylic acid,

6-(3-carboxyphenylacetylamino)-2α-methoxy-2-β-methylpenam-3-carboxylic acid,

6-(3-carboxyphenylacetylamino)-2α-methoxypenam-3-carboxylic acid,

6-(3-carboxyphenylacetylamino)-2α-ethoxypenam-3-carboxylic acid,

6-(3-carboxyphenylacetylamino)-2α-allenylpenam-3-carboxylic acid,

6-(3-carboxyphenylacetylamino)-2α-allenyl-2β-methylpenam-3-carboxylic acid, and

6-(3-carboxyphenylacetylamino)-2α-methoxy-2β-(n-propyl)penam-3-carboxylic acid.

In one aspect of the invention there is provided a process for preparing a 6-(3-carboxyphenylacetylamino)penam-3-carboxylic acid represented by the formula

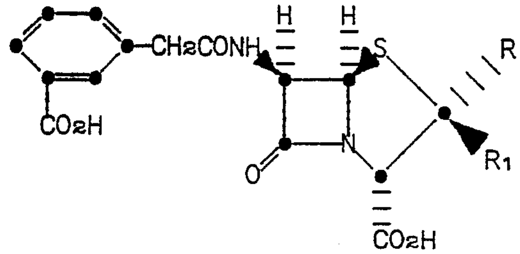

wherein R and $R_1$ independently are hydrogen or $C_1$-$C_4$ alkyl; or one of R and $R_1$ is hydrogen or $C_1$-$C_4$ alkyl and the other is $C_1$-$C_3$ alkoxy, $C_2$-$C_4$ alkenyl or allenyl. The process comprises contacting in an aqueous medium at a pH of between about 6 and about 9 in the presence of oxygen, ferrous ion and ascorbic acid, a dipeptide represented by the formula 2,

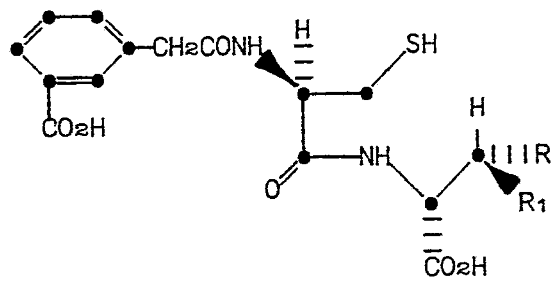

wherein R and $R_1$ have the above defined meanings, with the enzyme isopenicillin N synthetase.

In a second aspect of the invention N-(3-carboxyphenylacetyl)-L-cysteinyl-D-β,γ-didehydrovaline is incubated with the IPNS enzyme to form 7-(3-carboxyphenylacetylamino)-3-exomethylenecepham-4-carboxylic acid as illustrated in the following reaction scheme.

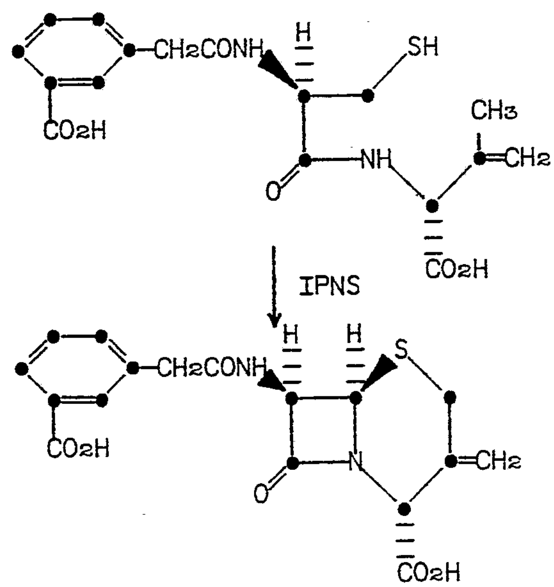

The 3-exomethylenecepham-4-carboxylic acid provided by the process is a useful intermediate to known 3-alkoxy-3-cephem and 3-halo-3-cephem antibiotics. For example, the product can be esterified to protect the carboxy groups and the diester N-deacylated by the method described by U.S. Patent No. 3,697,515 to provide, following deesterification, the 3-exomethylenecepham nucleus. This nucleus is described by U.S. Patent No. 3,932,393 and is represented by the formula

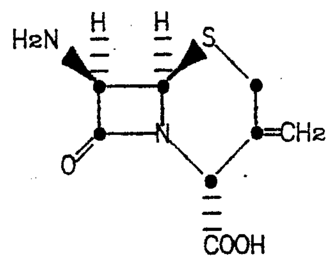

The 3-exomethylenecepham nucleus can be converted to the 3-methoxy-3-cephem antibiotics by procedures described by U.S. Patent 3,917,588 and to the 3-halo-3-cephem antibiotics by methods described by U.S. Patent 3,925,372.

Alternatively, the products of the process can be converted to the 3-halo-3-cephem or 3-methoxy-3-cephem derivatives and then N-deacylated to provide the known 3-halo-3-cephem and 3-methoxy-3-cephem nuclei. The latter can be reacylated to provide the desired 7β-acylamino derivative.

The 2-vinyl-substituted penam-3-carboxylic acid represented by the formula 1 can be converted to other 6-acylaminopenam antibiotics. For example, the

2-vinyl compound of formula 1 can be esterified to protect the 3-carboxy and 3-carboxyphenyl groups and the diester deacylated by known procedures to the 6-amino-2-vinylpenam-3-carboxylic acid ester. The latter nucleus ester can be N-acylated with the desired carboxylic acid and the acylated ester deesterified to provide the antibiotic. For example the 6-amino-2-vinylpenam ester is acylated with an active carboxy derivative of an amino-protected D-phenylglycine to provide, after removal of the amino-protecting group and the carboxy ester group, 6β-(D-phenylglycylamino)-2-vinylpenam-3-carboxylic acid. In general the N-acylation of the 6-amino-2-vinylpenam-3-carboxylic acid ester is carried out by employing the acylation methods used to N-acylate 6-aminopenicillanic acid or esters thereof.

The 2-alkoxypenam represented by the formula 1 is obtained with the dipeptides, 2, formed with methoxylated α-amino alkanoic acids and an S-protected N-(3-carboxyphenylacetyl) L-cysteine. Such dipeptides are formed with D-serine O-methyl ether D-serine-O-ethyl ether, 2R,3R-2-amino-3-methoxybutanoic acid, 2R,3R-2-amino-3-methoxypentanoic acid and like methoxylated alkanoic acids. Such amino acids are described in European Patent Application No. 85305911.1 (EPO Publication No. 0,174,129). For example, the benzyl ester of 2-amino-3-methoxybutanoic acid is coupled with an amino-protected S-(4-methoxybenzyl) cysteine with 2-ethoxy-N-ethoxycarbonyl-1,2-dihydroquinoline (EEDQ) to form the amino-protected ester dipeptide. The amino

protecting group is removed and the cysteinyl free amino group is acylated with the 3-(protected-carboxy)phenylacetic acid. The protecting groups are removed to provide the substrate 2 represented by the formula 2 wherein R is methyl and $R_1$ is methoxy.

Alternatively an S-protected ester of cysteine is acylated with the 3-(protected-carboxy)phenylacetic acid by known methods, the protecting group of the cysteinyl carboxy group is removed and the acid coupled via EEDQ (2-ethoxy-N-ethoxycarbonyl-1,2-dihydroquinoline) mediated coupling with the alkoxylated α-aminoalkanoic acid ester.

The compound 2 wherein $R_1$ is allenyl is prepared in a similar manner with the amino dienoic acid ester e.g. 2-amino-4,5-hexadienoic acid diphenylmethyl ester.

The following Preparations and Examples are provided to further illustrate the invention.

Preparation of
N-(3-carboxyphenylacetyl)-L-cysteinyl-D-valine

A. iso-Phthalic acid monobenzyl ester

To a suspension of iso-phthalic acid (11.2 g, 0.1 mole) in methyl alcohol (200 ml) and $H_2O$ (10 ml) was added a solution of potassium hydroxide (11.2 g, 0.2 mole) in methyl alcohol (100 ml), and the mixture was stirred at room temperature overnight. The solvent was removed under reduced pressure and DMF (250 ml) and

benzyl bromide (13.5 ml, 1.1 g) were added. The mixture was heated for 2 hours at 100°C, poured into aqueous sodium bicarbonate (10 g in 500 ml of water) and extracted with ethyl acetate. The aqueous layer was acidified (concentrated HCl) and extracted with ethyl acetate and the precipitated iso-phthalic acid was removed by filtration. The ethyl acetate layer was washed with brine, dried ($Na_2SO_4$) and evaporated. The residue (monobenzyl ester and iso-phthalic acid) was chromatographed on silica gel to give the monobenzyl ester 3.73 g (15%).

$^1H$ NMR (300 MHz) δ in $CDCl_3$: 5.42 (2H, s), 7.30-7.50 (5H, m), 7.59 (1H, t, J = 7.7 Hz), 8.32 (2H, t, J = 4.5 Hz), 8.81 (1H, s).

IR ($CHCl_3$): 2700-3400 (COOH), 1720 (s), 1700 (s) $cm^{-1}$.

B. 2,2,2-Trichloroethyl 3-(benzyloxycarbonyl)-phenylacetate

A mixture of iso-phthalic acid monobenzyl ester (2.6 g), dry benzene (8 ml) and pure thionyl chloride (2.6 ml, 3 eq.) was heated for 1 hour at 80°C. The solvent was removed under reduced pressure and then 10 ml of dry benzene were added and the solvent was again removed under reduced pressure in order to remove traces of unreacted thionyl chloride. The acid chloride was dissolved in dry benzene (20 ml) and the solution was poured into an ethereal solution of diazomethane (excess) at 0°C. The solution was stirred for 20

minutes at 0°C and then overnight at room temperature. The solvent and excess diazomethane were removed under reduced pressure. To the diazoketone was added 12 ml of 2,2,2-trichloroethanol and to the warm mixture (50 ~ 60°C) was added 0.3 g of silver oxide. Nitrogen was evolved and after 2 hours, 0.3 g of additional silver oxide was added and heating was continued for 30 minutes. A further 0.2 g of silver oxide was added and after 30 minutes, the diazoketone was consumed (by TLC). The mixture was diluted with chloroform, treated with charcoal, filtered and concentrated by evaporation. The residue was purified by flash chromatography [benzene-petroleum ether (1:1)]. There were obtained 3.6 g of the 2,2,2-trichloroethyl ester (88% yield). The diazomethane used was obtained with N-nitrosomethylurea.

$^1H$ NMR ($CDCl_3$, 300 MHz): δ 3.83 (2H, s), 4.76 (2H, s), 5.37 (2H, s), 7.30-7.60 (7H, m), 8.04 (2H, m).

IR ($CHCl_3$): 3020 (m), 2960 (w), 1755 (s), 1740 (s), 1610 (w), 1590 (w), 1500 (w), 1450 (m), 1375 (m), 1280 (s) $cm^{-1}$.

MS m/e: 91 (100), 225 [22, M- ($Cl_3CH_2OC(O)$], 293 [45, M- Ph($H_2O$)], 295 (42), 297 (14), 400 (7.7, $M^+$ $^{35}Cl_3$), 402 (6.9 M + 2), 404 (2.8 M + 4).

C. 3-(Benzyloxycarbonyl)phenylacetic acid

Zinc dust (7.2 g), followed by 1M aqueous potassium dihydrogen phosphate ($KH_2PO_4$) (7.2 ml) were added to a rapidly stirred solution of the 2,2,2-trichloroethyl ester prepared as described above (3.6 g) in tetrahydrofuran (36 ml) at room temperature. After 15 minutes TLC showed no reaction had occurred. A further 7.2 g of zinc were added. After a few minutes the temperature had increased. After 30 minutes the zinc was filtered off and the solvent was removed under reduced pressure. The residue was dissolved in chloroform and 2N HCl (20 ml) was added and the mixture was stirred for 1 hour. The chloroform layer was separated and the aqueous layer was extracted with chloroform. The extract was combined with the chloroform layer, washed with brine, dried over sodium sulfate and evaporated. The residue was purified by flash chromatography (ethyl acetate) to give 869 mg (37% yield) of 3-(benzyloxycarbonyl)phenylacetic acid.

m.p. 95-96° (from ether)
Elemental analysis calculated for $C_{16}H_{14}O_4$:
Theory: C, 71.10; H, 5.22
Found: C, 71.22; H, 5.27

$^1$H NMR (300 MHz, $CDCl_3$): δ 3.72 (2H, s), 5.37 (2H, s), 7.30-7.55 (7H, m), 7.98-8.09 (2H, m).

MS m/e: 91 (34), 163 [100, M- Ph($H_2O$)], 252 (6.7, M- $H_2O$), 270 ($M^+$, 10).

IR ($CHCl_3$): 2800 ∿ 3400 (COOH), 1715 (C = O) $cm^{-1}$.

D. Acylation of S-(4-methoxybenzyl)-L-cysteinyl-D-valine diphenylmethyl ester

A mixture of 3-(benzyloxycarbonyl)phenylacetic

acid (427 mg, 1.58 mmole), EEDQ (390 mg, 1 equiv.) and S-(4-methoxybenzyl)-L-cysteinyl-D-valine benzhydryl ester (1 equiv.) in 10 ml of dry methylene chloride was stirred at room temperature overnight. The solvent was evaporated and the residue was dissolved in 80 ml of ethyl acetate. The solution was washed with aqueous sodium bicarbonate, brine, 10% aqueous citric acid, again with brine and was dried over sodium sulfate. The solvent was evaporated and the residue purified by flash chromatography (20% ethyl acetate-petroleum ether). There were obtained 1.005 g of N-[3-(benzyloxycarbonyl)phenylacetyl]-S-(4-methoxybenzyl)-L-cysteinyl-D-valine diphenylmethyl ester melting at about 136°C to about 137°C (diethyl ether).

Elemental analysis calculated for $C_{45}H_{46}N_2O_7S$:
Theory: C, 71.22; H, 6.11; N, 3.69
Found: C, 71.12; H, 6.02; N, 3.59

$^1H$ NMR ($CDCl_3$) (300 MHz): δ 0.73 and 0.85 (each 3H, d, J = 6.9 Hz), 2.23 (1H, m), 2.62 (1H, dd, J = 14.0, 7.5 Hz), 2.82 (1H, dd, J = 14.0, 5.4 Hz), 3.56 (2H, s), 3.69 (2H, s), 3.76 (3H, s), 4.52 (1H, dd, J = 7.5, 5.4 Hz), 4.63 (1H, dd, J = 8.5, 4.4 Hz), 5.35 (2H, s), 6.35 (1H, d, J = 7.5 Hz), 6.70 (1H, d, J = 8.5 Hz), 6.83 and 7.16 (each 2H, d, J = 8.0 Hz), 6.91 (1H, s), 7.28 ∿ 7.48 (17H, m), 7.96 (2H, m).

IR ($CHCl_3$): 3400, 3010, 2960, 1720, 1670, 1610, 1515, 1500 $cm^{-1}$.

MS: 758 ($M^+$).

The triprotected N-acylation product (150 mg, 0.2 mmole) was dissolved in 3 ml of methylene chloride and 0.36 ml of anisole was added. The solution was cooled in an ice bath and 2 ml of a 0.5M solution of aluminum chloride in nitromethane was added. The mixture was stirred for 2 hours at 0°C and then for 3 hours at room temperature. The reaction mixture was diluted with ethyl acetate, washed with 1N hydrochloric acid and extracted with 5% aqueous sodium bicarbonate. The extract was washed with ethyl acetate, acidified with hydrochloric acid and the deprotected peptide extracted with ethyl acetate. The extract was washed with brine, dried and evaporated to provide 70 mg (93% yield) of the title compound melting at about 196°C to about 197°C (decomposition) (from acetone).

Analysis calculated for $C_{17}H_{22}N_2O_6S$:

Theory: C, 53.39; H, 5.80; N, 7.32

Found: C, 53.55; H, 5.82; N, 7.23

$^1H$ NMR (300 MHz, $CD_3OD$): δ 0.89 and 0.94 (each 3H, d, J = 6.9 Hz), 2.15 (1H, m), 2.81 and 2.90 (each 1H, dd, J = 14.0, 6.5 Hz), 3.67 (2H, s), 4.31 (1H, d, J = 5.4 Hz), 4.58 (1H, t, J = 6.5 Hz), 7.42 (1H, t, J = 7.7 Hz), 7.56 (1H, d, J = 7.7 Hz), 7.90 (1H, d, J = 7.7 Hz), 8.01 (1H, s).

MS (FAB EX. glycerol/oxalic acid) m/e: 383 (M + 1).

IR (KBr): 3300, 2800 ∿ 3300 (broad), 2680 (w), 2560 (w), 1730, 1700, 1650, 1605 $cm^{-1}$.

Preparation of D-diphenylmethyl 2-amino-4,5-hexadienoate p-toluenesulfonate

N-[(1,1-Dimethylethoxy)carbonyl]-3-iodo-D-alanine diphenylmethyl ester (481 mg), triphenyl propargyl tin (780mg) and azobisisobutyronitrile (AIBN) (0.15eq) were dissolved in dry degassed benzene (2ml) and the solution was refluxed under nitrogen at 80° for 18h. Fresh quantities of tin reagent (1 eq) and AIBN (10mg) were added after 4, 8 and 12h. The reaction mixture was cooled, evaporated to dryness, and the residue taken up in acetonitrile, washed with hexane then evaporated to leave an oily residue which was flash chromatographed over flash silica gel; dichloromethane eluates gave the title compound (247mg) crystallized from petroleum ether-dichloromethane m.p. 53°.

A solution of anhydrous p-toluenesulfonic acid (15 mg) in ethanol was added slowly (15 min) to an ether solution of the amino acid ester (34 mg) at -5° to -10°. The solution was stirred at room temperature under argon for 3h then evaporated to dryness to leave the title compound (40 mg) m.p. 128-130°.

Preparation O-Methyl-D-serine benzyl ester, hydrochloride N-p-Methoxybenzyloxycarbonyl-D-serine benzyl ester

N-p-Methoxybenzyloxycarbonyl-D-serine (5.39 mg, 2.00 mmol; prepared as a colourless solid of m.p. 96-97°C in 60% yield by the procedure of Waygand and Hunger, Berichte, 1962, 95 (1), 1) is dissolved in

methanol and treated with a solution of potassium carbonate (138 mg, 1.00 mmol) in water (2 ml). The mixture is evaporated by dryness, N,N-dimethyl-formamide (DMF) is added and re-evaporated (2 x 2 ml), and the solid residue is then dissolved in dry warm DMF, benzyl bromide (248 µl, 2.1 mmol) is added and the solution is warmed in a dry closed flask at 50C for 1 hour. The resulting mixture is partioned between ether (10 ml) and water (10 ml) and the aqueous layer is extracted with ether (2 x 10 ml). The combined ether extracts are washed with saturated sodium bicarbonate (5 ml) and saturated brine (5 ml), dried and evaporated to dryness. The residue is purified on a column of silica gel using 1:1 v/v ethyl acetate:petroleum ether as eluant to yield to title compound as a homogenous oil (590 mg, 82%) which crystallises on standing to give a colourless solid of m.p. 67-68°C (from diethyl ether); $[a]_D^{20}$ -6.3° (c 4.0, $CHCl_3$).

N-p-Methoxybenzyloxycarbonyl-O-methyl-D-serine benzyl ester

N-p-Methoxybenzyloxycarbonyl-D-serine benzyl ester (720 mg, 2.00 mmol) is dissolved in dry dichloro methane (20 ml) and the solution cooled in dry ice/-acetone. Boron trifluoride etherate (100 µl) is added followed by the portionwise addition of diazomethane (30 mmol in 20 ml $CH_2Cl_2$). After about 30 minutes the solution is filtered and washed once with water (10 ml), then dried ($MgSO_4$) and evaporated to dryness. The

resulting residue is chromatographed on silica gel using chloroform as eluant to give the pure title compound as an oil (520 mg, 70%) which crystallises on standing to give a colourless solid of m.p. 68-69°C; $[\alpha]_D^{20}$ -1.3° (c 4.0, $CHCl_3$).

O-Methyl-D-serine benzyl ester, hydrochloride

To N-p-methoxybenzyloxycarbonyl-O-methyl-D-serine benzyl ester (130 mg, 0.35 mmol) is added a mixture of cold trifluoroacetic acid (TFA): anisole (4.8:1 v/v) (0.6 ml). The mixture is shaken on an ice bath until all the solid has dissolved and the TFA is then evaporated initially on a rotary evaporator at 0°C, finally using a high vacuum pump. The resulting residue is purified by chromatography on silica gel using 98:2 v/v chloroform:methanol as eluant to give the title compound in basic form as a yellowish oil (38 mg, 55%); this is converted to the hydrochloride salt which is a white solid, m.p. 100-101°C. $[\alpha]_D^{20}$ +15.5° (c 2.0, $CH_3OH$).

Preparation of 2R,3R- and 2S,3S-2-Bromo-3-methoxybutanoic acid benzyl ester

Crotonic acid (8.6 g, 100 mmol) is dissolved in methanol (50 ml) and N-bromacetamide (13.8 g, 10 mmol) is added in portions over 30 minutes. The solution is stirred at 25°C for 15 hours and the solvent is then evaporated and the residue partitioned between diethyl ether and water. The ether layer is dried

($Na_2SO_4$), filtered, evaporated and the residue distilled to yield the title compounds as a colourless oil (14.3 g, 72%), b.p. 88-89°/0.5 mm.

Preparation of 2R,3R- and 2S,3S-2-Amino-3-methoxybutanoic acid

2R,3R- and 2S,3S-2-Bromo-3-methoxybutanoic acid (14 g, 71 mmol) are dissolved in ammonia (e.g. 0.88, 250 ml) and the mixture heated in an autoclave at 95°C for 8 hours. The mixture is cooled to 25°C, evaporated, and the residue suspended in acetone. The resultant colourless solid is filtered off and washed with acetone to give the title compounds as a colourless solid containing residual ammonium bromide, (12.1 g) m.p. 180-184°C (dec.).

Preparation of 2R,3R- and 2S,3S- N-Benzyloxycarbonyl-2-amino-3-methoxybutanoic acid, benzyl ester

2R,3R- and 2S,3S-2-Amino-3-methoxybutanoic acid (3.1 g) are dissolved in a mixture of 1M sodium hydroxide (27 ml), water (20 ml), and dioxan (40 ml). Benzyl chloroformate (3.8 ml, 22 mmol) in dioxan (20 ml) and 1M sodium hydroxide (27 ml) are added to the solution individually, each at the same rate of addition, over 25 minutes. The mixture is stirred for 1 hour, extracted into ethyl acetate (3 x 200 ml), acidified to pH 1 with 2N hydrochloric acid and then re-extracted into ethyl acetate (3 x 200 ml). The combined organic

extracts are dried, filtered, and evaporated to give an oil. This oil is dissolved in dry dimethyl formamide (20 ml) and to the solution are added sodium hydrogen carbonate (3.14 g, 41 mmol), benzyl bromide (3.8 ml, 33 mmol), anhydrous sodium sulphate (200 mg) and sodium iodide (10 mg), the whole being stirred at 25°C for 24 hours. The mixture is then extracted into dichloromethane (200 ml), washed with water (4 x 300 ml), dried (sodium sulphate), filtered and evaporated. Purification by chromatography on silica gel using ethyl acetate and petroleum ether as consecutive eluants gives the title compounds as a colourless oil (4.2 g, 65%) which solidifies on standing, m.p. 44°C.

Preparation of 2R,3R- and 2S,3S-2-Amino-3-methoxybutanoic acid, benzyl ester

2R,3R- and 2S,3S-N-Benzyloxycarbonyl-2-amino-3-methoxybutanoic acid, benzyl ester (500 mg, 1.4 mmol) are dissolved in dry dichloromethane (3 ml) and hydrobromic acid (45% in acetic acid, 2 ml) is added. The mixture is stirred under argon for 20 minutes and is then evaporated. The residue is dissolved in dichloromethane (3 x 3 ml) and re-evaporated (3x) to give a further residue which is dissolved in xylene (2 x 3 ml) and re-evaporated (2x). Petroleum ether (5 ml) is then added and the product is triturated for 5 minutes before discarding the mother liquor and dissolving the solid residue in dichloromethane (5 ml). Triethylamine (1 ml) is added to the dichloromethane solution which is then

stirred for 2 minutes and evaporated. The residue is dissolved in dichloromethane (3 x 5 ml) and re-evaporated (3x) to give a further residue which is triturated with diethyl ether (5 ml). The resulting solid is filtered off, re-extracted with diethyl ether (2 x 5 ml) and the ethereal layers combined and evaporated to yield the title compounds as a colourless oil (290 mg, 93%).

Preparation of benzyl 2-amino-3-methylpent-4-enoate formic acid salt

Benzyl 2-(t-butyloxycarbonylamino)-3-methyl pent-4-enoate

A mixture of 4.4 mmol of 2-(t-butyloxycarbonylamino)-3-methylpent-4-eneoic acid (2R,3S; 3S,2R) prepared by the method of P. A. Bartlett et al., J. Org. Chem., 1982, 47, 3933, in 10 ml of dry DMF containing 4.4 mmol of benzyl bromide, 4.4 mmol of sodium bicarbonate and 10 mg of sodium iodide was stirred for 15 minutes at 20°C. The mixture was dissolved in ethyl acetate, the solution washed three times with water, was dried over sodium sulfate, filtered and evaporated. The ester product was purified by flash chromatography on silica gel. The ester was obtained in an 80% yield as an oil.

NMR (300 MHz, $CDCl_3$): δ 1.01 (3H, d, J 6Hz, 3-$CH_3$), 1.44 (9H, s, t-butyl), 2.58-2.71 (1H, m, 3-H), 4.32-4.40 (1H, m, 2-H), 5.01-5.27 (5H, m, 5-H, NH,

$CH_2C_6H_5$), 5.05-5.37 (1H, m, 4-H), 7.33-7.41 (5H, m, $C_6H_5$-H).

IR ($CHCl_3$ solution): 3005 m, 1730 s ($CO_2$), 1710 s ($CO_2$), 1600 w, 1502 m, 1205 s, 1160 m $cm^{-1}$.

m/e ($NH_3$, Desorption Chemical Ionization): 320 ($MH^+$, 100%)

Benzyl 2-amino-3-methylpent-4-eneoate formic acid salt

The benzyl ester, 0.40 mmol, was dissolved in 1 ml of formic acid and the solution stirred for 2 hours at 20°C. The solution was evaporated to yield the crude formic acid salt.

## Example 1

Enzymatic Cyclization of N-(3-carboxyphenylacetyl)-L-cysteinyl-D-valine

A mixture of N-(3-carboxyphenylacetyl)-L-cysteinyl-D-valine (1.4 mg, 3.66 x $10^{-3}$ mmole), 100 µl of 100 mmolar dithiothreitol, 100 µl of 5 mmolar ferrous sulfate, 100 µl of 50 mmolar L-ascorbic acid, 50 µl of catalase (1/10 dilution), 1 ml of 50 mmolar ammonium bicarbonate and 3.5 ml of an isopenicillin N-synthetase solution in 50 mmolar ammonium bicarbonate [prepared from 1 ml of IPNS TRIS·HCl buffer (activity 2700 C.U. $ml^{-1}$, specific activity of 109.8 C.U. $ml^{-1}$)] was divided into two equal volumes and each half placed in an open

vial. The contents of each vial were incubated at 30°C (open to air) at 250 rpm. After 30 minutes 50 µl of 100 mmolar dithiothreitol and 50 µl of 5 mmolar ferrous sulfate were added to each vial and incubation was continued for 30 minutes. The incubated mixtures were combined, 12 ml of acetone added and the mixture centrifuged. The supernatant was decanted and evaporated to dryness and the residue of product was freeze dried. The product was purified via reverse phase $C_{18}$ analytical column using 3% acetonitrile-90% 10 mmolar ammonium bicarbonate.

$^1H$ NMR (300 MHz, $D_2O$): δ 1.30 (3H, s), 1.38 (3H, s), 3.55 and 3.63 (each 1H, d, J = 15.0 Hz), 4.07 (1H, s), 5.25 and 5.35 (each 1H, d, J = 3.8 Hz), 7.35 (2H, m), 7.71 (2H, m).

The product was subjected to NMR examination with sodium trimethylsilyl propionate-$d_4$ ($1.83 \times 10^{-4}$ mmole) for the determination of β-lactam. The yield of product, 6β-(3-carboxyphenylacetylamino)penicillanic acid, was 68%.

## Example 2

6β-(3-Carboxyphenylacetylamino)-2α-vinyl-2β-methylpenam-3-carboxylic acid is obtained with the substrate N-(3-carboxyphenylacetyl)-L-cysteinyl-D-γ,δ-didehydroisoleucine by the method of Example 1.

Example 3

6β-(3-Carboxyphenylacetylamino)-2α-methoxy-2β-methylpenam-3-carboxylic acid is obtained with the substrate N-(3-carboxyphenylacetyl)-L-cysteinyl-(2R,3R-2-amino-3-methoxybutanoic acid by the method of Example 1.

Example 4

7β-(3-Carboxyphenylacetylamino)-3-exomethylene-cepham-4-carboxylic acid is obtained with the substrate N-(3-carboxyphenylacetyl)-L-cysteinyl-D-β,γ-didehydro-valine by the method of Example 1.

Example 5

6β-(3-Carboxyphenylacetylamino)-2α-allenyl-penam-3-carboxylic acid is obtained with the substrate N-(3-carboxyphenylacetyl)-L-cysteinyl-(2R-2-amino-4,5-hexadienoic acid) by the method of Example 1.

## Claims

1. A process for preparing a β-lactam of the formula:

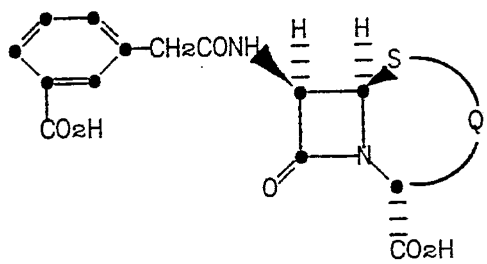

where Q represents a group of formula:

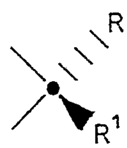

or

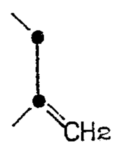

and

wherein R and $R_1$ independently are hydrogen or $C_1$-$C_4$ alkyl; or one of R and $R_1$ is hydrogen or $C_1$-$C_4$ alkyl and the other is $C_1$-$C_3$ alkoxy, $C_2$-$C_4$ alkenyl or allenyl; which comprises contacting in an aqueous medium at a pH of between about 6 and about 9 in the presence of oxygen, ferrous ion and ascorbic acid, a dipeptide of the formula:

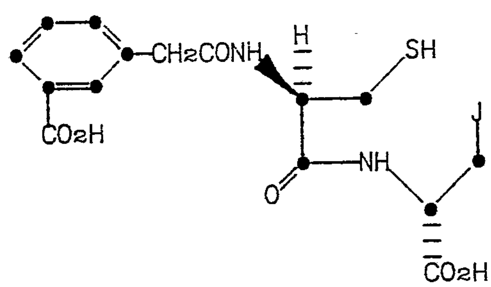

where J represents

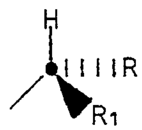

or

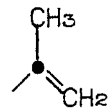

and wherein R and $R_1$ have the above defined meanings, with the enzyme isopenicillin N synthetase.

2. A process according to claim 1 for preparing a 6-(3-carboxyphenylacetylamino)penam-3-carboxylic acid of the formula:

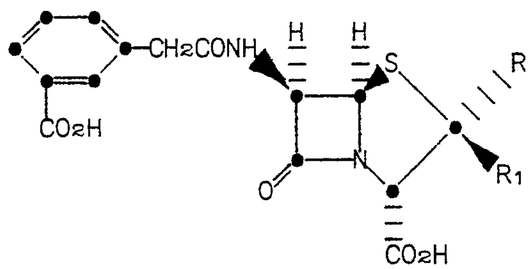

wherein R and $R_1$ independently are hydrogen or $C_1$-$C_4$ alkyl; or one of R and $R_1$ is hydrogen or $C_1$-$C_4$ alkyl and the other is $C_1$-$C_3$ alkoxy, vinyl or allenyl which comprises contacting a dipeptide of the formula:

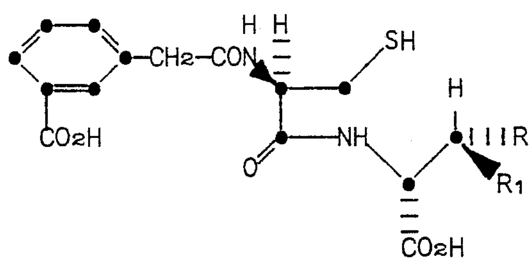

, with the enzyme isopenicillin N synthetase.

3. A process according to claim 2 wherein R and $R_1$ are methyl.

4. A process according to claim 1 for preparing a 3-exomethylenecepham-4-carboxylic acid of the formula:

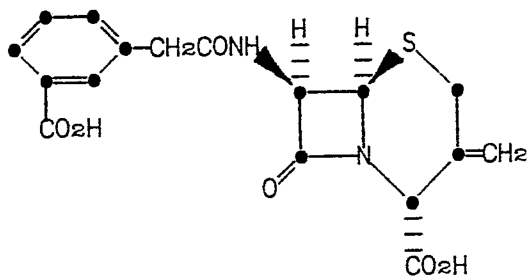

which comprises contacting a dipeptide of the formula:

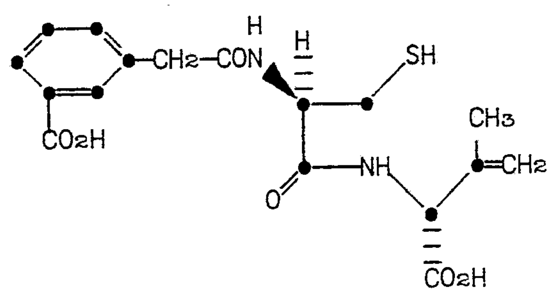

with the enzyme isopenicillin N synthetase.

5. The compound of the formula:

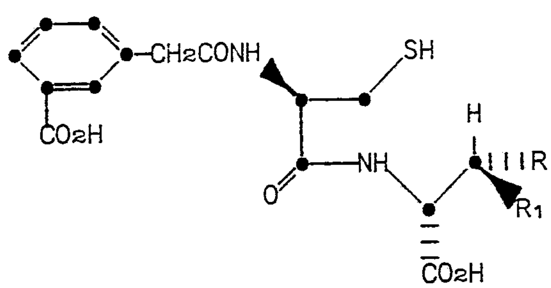

wherein R and $R_1$ independently are hydrogen or $C_1$-$C_4$ alkyl; or one of R and $R_1$ is hydrogen or $C_1$-$C_4$ alkyl and the other is $C_1$-$C_3$ alkoxy, $C_2$-$C_4$ alkenyl or allenyl.

6. A compound of claim 5 wherein R and $R_1$ are methyl.

7. A compound of claim 5 wherein one of R and $R_1$ is hydrogen or $C_1$-$C_4$ alkyl and the other is vinyl.

8. The compound of the formula:

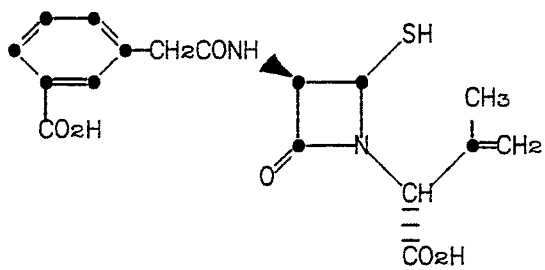

0260778

European Patent Office

# EUROPEAN SEARCH REPORT

Application Number

EP 87 30 1254

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | CHEMICAL ABSTRACTS, vol. 106, no. 11, 16th March 1987, page 477, abstract no. 83040u, Columbus, Ohio, US; & ES-A-545 213 (ANTIBIOTICOS S.A.) 16-02-1986 * Abstract * --- | 1 | C 12 P 37/00<br>C 12 P 35/00<br>C 07 K 5/06 //<br>C 12 R 1/465<br>C 12 R 1/75<br>C 12 R 1/82 |
| Y | CHEMICAL ABSTRACTS, vol. 107, no. 9, 31st August 1987, page 563, abstract no. 76081a, Columbus, Ohio, US; & ES-A-548 662 (ANTIBIOTICOS S.A.) 16-03-1986 * Abstract * --- | 1 | |
| Y | CHEMICAL ABSTRACTS, vol. 107, no. 9, 31st August 1987, page 563, abstract no. 76082b, Columbus, Ohio, US; & ES-A-548 676 (ANTIBIOTICOS S.A.) 16-03-1986 * Abstract * --- | 1 | |
| Y | JOURNAL OF THE CHEMICAL SOCIETY CHEMICAL COMMUNICATIONS, no. 24, 1985, pages 1808,1809, The Royal Society of Chemistry; J.E. BALDWIN et al.: "Penicillin biosynthesis: direct biosynthetic formation of penicillin V and penicillin G" * Whole document * --- | 1 | TECHNICAL FIELDS SEARCHED (Int. Cl.4)<br>C 12 P 37/00<br>C 12 P 35/00<br>C 07 K 5/00 |
| Y | THE JOURNAL OF ANTIBIOTICS, vol. 39, no. 8, August 1986, pages 1144-1147; J.M. LUENGO et al.: "Cyclization of phenylacetyl-L-cysteinyl-D-valine to benzylpenicillin using cell-free extracts of Streptomyces clavuligerus" * Whole document * --- -/- | 1 | |

The present search report has been drawn up for all claims

| Place of search | Date of completion of the search | Examiner |
|---|---|---|
| THE HAGUE | 08-12-1987 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number

EP 87 30 1254

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | THE JOURNAL OF ANTIBIOTICS, vol. 39, no. 6, June 1986, pages 822-826; S.E. JENSEN et al.: "Synthesis of benzylpenicillin by cell-free extracts from Streptomyces clavuligerus" * Whole document * --- | 1 | |
| Y | CHEMICAL ABSTRACTS, vol. 105, no. 7, 18th August 1986, page 275, abstract no. 56843f, Columbus, Ohio, US; J.M. CASTRO et al.: "Conversion of phenylacetyl-cysteinyl-valine in vitro into penicillin G by isopenicillin N synthase of Streptomyces lactamdurans", & FEMS MICROBIOL. LETT. 1986, 34(3), 349-53 * Abstract * --- | 1 | |
| Y | JOURNAL OF THE CHEMICAL SOCIETY CHEMICAL COMMUNICATIONS, no. 18, 1984, pages 1225-1227; J.E. BALDWIN et al.: "Penicillin biosynthesis: active site mapping with aminoadipoylcysteinylvaline variants" * Whole document * --- | 1 | TECHNICAL FIELDS SEARCHED (Int. Cl.4) |
| A | US-A-4 307 192 (DEMAIN et al.) * Claims * --- | 1 | |
| A | EP-A-0 102 216 (QUEEN'S UNIVERSITY AT KINGSTON) * Claims * ----- | 1 | |

The present search report has been drawn up for all claims

| Place of search | Date of completion of the search | Examiner |
|---|---|---|
| THE HAGUE | 08-12-1987 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)